# EUROPEAN PATENT APPLICATION

(11) **EP 2 181 695 A2**
(43) Date of publication of application: **05.05.2010**
(21) Application number: 09013000.6
(22) Date of filing: 14.10.2009
(51) Int. Cl.: A61K 8/31, A61K 8/37, A61K 8/81, A61Q 1/14

(54) **Mineral-oil free, polymerically stabilized make-up remover**

(30) Priority: 15.10.2008 US 196309 P
(71) Applicant: L'Oréal, 75008 Paris (FR)
(72) Inventor: Russell, Michael, Chatham, New Jersey 07928 (US); Cornell, Marc, Jackson, New Jersey 08527 (US)
(74) Representative: Blodig, Wolfgang

(57) **Abstract**

Disclosed are make-up removal compositions, comprising a mineral oil-free oil phase; a sodium salt of an acrylate copolymer; a stabilizing polymer; and a hydrophobic wetting agent, and methods of using the compositions to remove make-up from facial tissue or skin. The disclosed compositions exhibit a rheological profile that features a variable ("up and down" or "increasing and then decreasing") shear stress response to increasing shear rates, and which is believed to contribute to better performance than known make-up removal compositions tested.

## Description

### BACKGROUND OF THE INVENTION

A variety of compositions are known which function to remove make-up from skin, particularly facial tissue. U.S. Patent 6,200,579, for example, teaches a composition consisting of an aqueous phase and an oily phase, which are distinct, to remove the make-up or transfer-free make-up. U.S. Patent 6,342,469 teaches make-up removing and/or cleansing cosmetic composition in the form of a water-in-oil emulsion and comprising (1) at least one silicone emulsifier, (2) at least one branched-chain hydrocarbonaceous oil and (3) at least one make-up, removing oil chosen from esters of a fatty acid comprising at least 12 carbon atoms. U.S. Patent 6,428,775 teaches a cosmetic make-up removal composition consisting essentially of: (i) about 15 to about 25% of a first make-up removing ingredient selected from the group consisting of waxes, mineral oil and mixtures thereof, (ii) at least about 5% of an isoparaffin as a second make-up removing ingredient; and (iii) a cosmetically acceptable vehicle.

### SUMMARY OF THE INVENTION

A first aspect of the present invention is directed to a make-up removal composition, comprising a mineral oil-free oil phase; a sodium salt of an acrylate copolymer, such as a sodium acrylates copolymer; a polymeric stabilization agent; a hydrophobic wetting agent; and an aqueous phase.

Another aspect of the present invention is directed to a method of removing make-up from facial tissue or skin, comprising applying to facial tissue a make-up removal composition, comprising a mineral oil-free oil phase; a sodium salt of an acrylate copolymer; a polymeric stabilization agent; and a hydrophobic wetting agent; and an aqueous phase.

As shown in working examples below which describe consumer performance testing, the present invention was found to provide superior make-up removal capabilities compared to other make-up removal compositions, including commercially available compositions and compositions that have similar and dissimilar ingredients. Without intending to be bound by theory, Applicants attribute the unexpectedly superior performance of the inventive compositions to a unique "break and reform" rheological profile. As described in the working examples and illustrated in figures herein, the "break and reform" profile features successive cycles of a decrease and then increase in viscosity during application (e.g., rubbing) to facial tissue and removal of the make-up from facial tissue. Stated somewhat differently, Applicants have discovered that in accordance with the "break and reform" rheology, the inventive compositions can break on the make-up, removing a quantity of make-up, and then reform and do the same process over again until all the make-up is lifted off the facial tissue or skin. Based on the results of the experiments described in the working examples, it was observed that unlike known make-up removal compositions, an inventive composition performed the "break and reform" rheology rather quickly in make-up removal timings and left little to no residue on the skin.

As illustrated in the figures and explained in detail herein, the inventive make-up removal compositions exhibit a variable ("up and down" or "increasing and then decreasing") shear stress response to increasing shear rates, e.g., at 20, 40, 80 and 150 1/seconds, when measured using a rheometer such as Prorheo Model R 180.

### BRIEF DESCRIPTION OF THE DRAWINGS

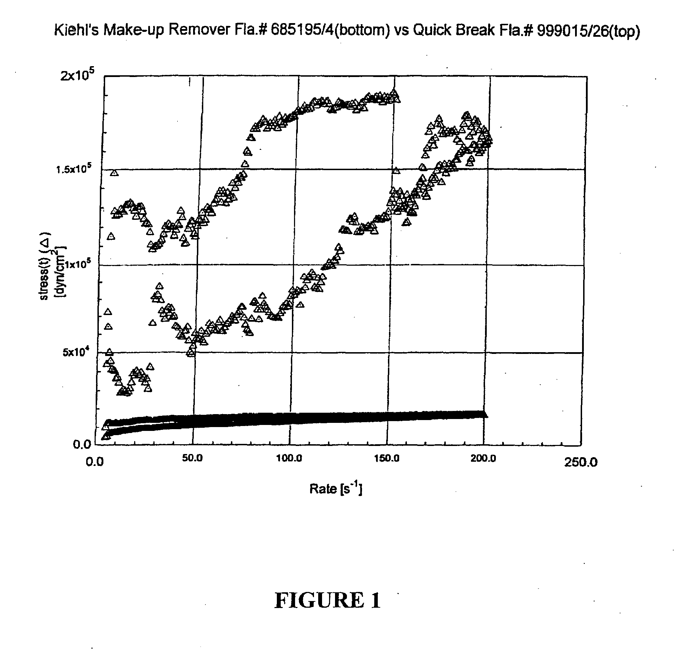

Fig. 1 is a graph (rheogram) that compares the rheological profiles of an embodiment of the present invention (described in example 1) to a commercial emulsion-type make-up remover In this type of rheological evaluation, the method entails applying increasing and decreasing shear rate ramps while recording changes in shear stress. Rheograms contain two different flow curves, one representing acceleration (the "up" curve) followed by another flow curve corresponding to deceleration (the "down" curve). These curves are generated during a complete testing cycle. This loop is often referred to in the art as the hysterisis loop and the area within the loop represents a measure of thixotropic structure of a formulation. A decrease in this loop area is a measure of the extent of the structural breakdown during a shear program. It is apparent that in the upper rheogram shown in this graph that the inventive composition generated a significantly larger loop area than the area generated by the commercial product (lower rheogram). More importantly, the larger (top) rheogram shows evidence of variable shear stress response to increasing shear rates. This is seen in the "up and down" curve response at 20, 40, 80 and 150 1/seconds. In sharp contrast, the comparative product did not produce any "up and down" variable response to changes in shear rate.

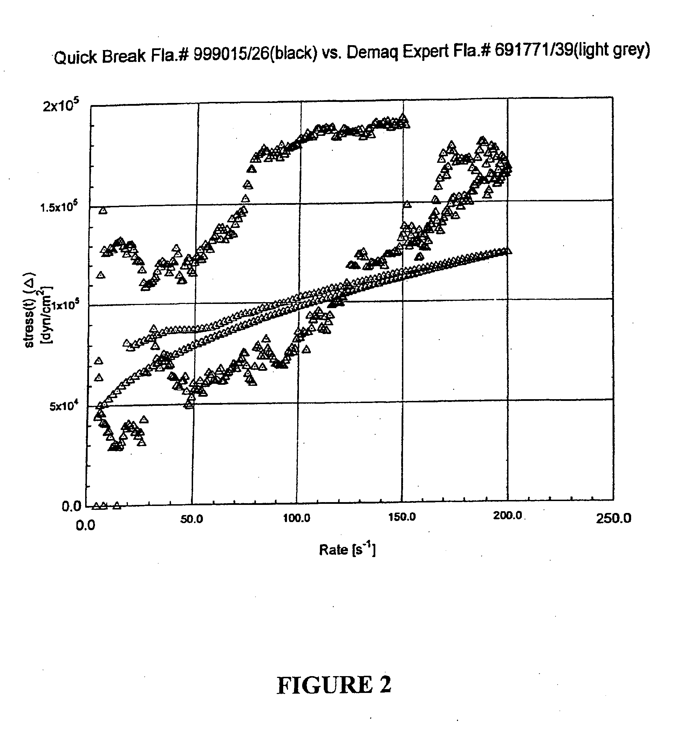

Fig. 2 is a graph that compares the rheological profiles and differences in thixotropic structure (area between the curves) of an embodiment of the present invention (described in example 1) to a make-up removal composition with similar ingredients. Despite these similarities, the rheological profiles are clearly different. There is no variable or "up and down" shear stress response in relation to changes in shear rate for the comparative composition.

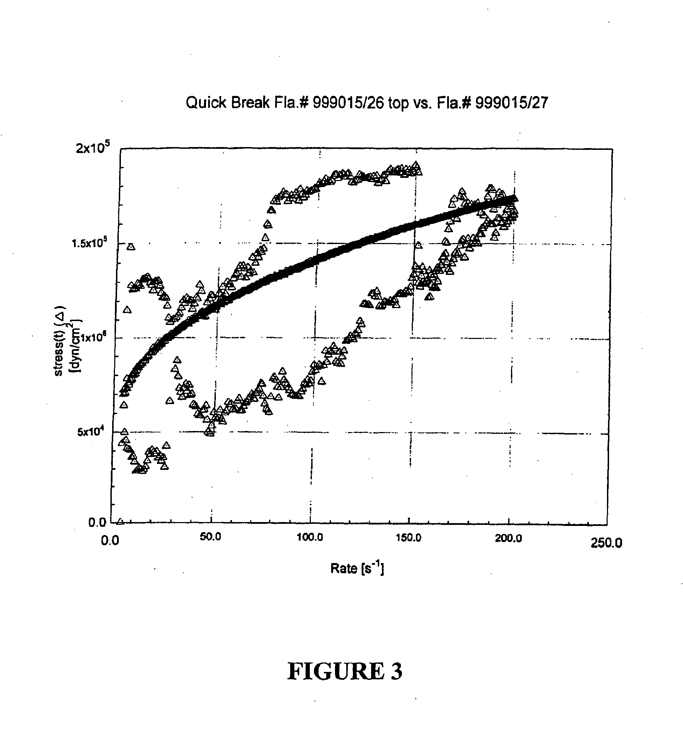

Fig. 3 is a graph that compares the rheological profiles and differences in thixotropic structure (area between the curves) of an embodiment of the present invention (described in example 1) to a make-up removal composition with similar ingredients. Despite these similarities, the rheological profiles are clearly different. There is no "up and down" shear stress response in relation to changes in shear rate for the comparative composition.

### DETAILED DESCRIPION OF THE INVENTION

The oil phase of the inventive compositions contains at least one oil. Oils that may be useful in the present invention include straight or branched chain hydrocarbons or esters thereof. The hydrocarbon chain is typically C12 to C26, and in some embodiments from C16 to C26. The esters include both lower and higher alkyl esters, e.g., C14 to C30. The polarity index of these hydrocarbons generally ranges from about 10 to about 40. Examples include isohexadecane, isopropyl myrstate, octyl palmitate, octyl stearate, ethylhexyl stearate and ethylhexyl palmitate oil. In some embodiments, the oil phase contains isohexadecane and isopropyl myrstate. Yet other examples of suitable oils include non-hydrocarbons known in the art as being useful in make-up removal, such as cyclopentasiloxane.

The amount of the oil phase in the inventive compositions generally varies between about 10 to about 45% by weight, and in some embodiments, from about 15 to about 30%, and in some embodiments from about 20% to about 30% and in some other embodiments from about 25 to about 28, 29 or 30% by weight of the composition.

Examples of sodium salts of acrylate (co)polymers include sodium polyethylacrylate, sodium polyacrylamide (commercially available from SEPPIC under the tradename SEPIGEL 305)), and sodium salts of acrylate copolymers. Acrylates (co)polymers useful in the present invention are more generally referred to in the art as acrylic film-forming dispersions as they are commercially available in the form of liquid dispersions or emulsions. Examples of acrylates copolymers (which can be in sodium salt form) are known in the art, and include ethyl acrylates/methyl methacrylates copolymer emulsion (chemical name)(INCI name:water (and) acrylates copolymer), which is commercially available from Kobo Products, Inc. (South Plainfield, NJ) and Daito Kasei Kogyo Co., Ltd., under the trade name Daitosol AD. This product is sold in the form of an emulsion that contains water, ethyl acrylates/methyl methacrylates copolymer, sodium dehydroacetate, and Laureth-20 (lauryl alcohol and oxirane). See, United States Patent Application Publication 20060134043 A1. Another suitable acrylates copolymer is ethyl methacrylates/N-butyl acrylates/2-methylhexyl acrylates copolymer emulsion (chemical name)(INCI name:water (and) acrylates/ethylhexyl acrylates copolymer), which is also commercially available from Kobo Products, Inc. and Daito Kasei Kogyo Co., Ltd., under the trade name Daitosol SJ. This product is sold in the form of an emulsion that contains water, ethyl methacrylates/N-butyl acrylates/2-methylhexyl acrylates copolymer, and Laureth-20. Other yet other acrylates copolymers include an alkyl (meth)acrylates copolymer emulsion (INCI name: acrylates copolymer), which is commercially available from Nippon LSC Ltd., under the trade name Yodosol GH34F; a styrene/acrylates copolymer emulsion (INCI name), which is commercially available from Nippon LSC Ltd., under the tradename Yodosol GH41F; a Polyacrylates-21 (and) acrylates/dimethylaminoethyl methacrylates copolymer (INCI name), commercially available from Interpolymer under the tradename Syntran 5100, the chemical composition of which includes, in addition to water and the two acrylates copolymers having CAS Nos. 68541-61-7 and 67380-24-9 respectively, ethoxylated secondary alcohol (CAS No. 84133-50-6) and sodium laurylpolyethoxyethanol sulfate (CAS No. 68891-38-3); a styrene/acrylates/ammonium methacrylates copolymer (and) butylene glycol (and) sodium Laureth-12 sulfate (INCI name), commercially available from Interpolymer under the tradename Syntran 5760 as a 40% aqueous dispersion; and a polyurethane-10 and PEG-12 dimethicone alcohol copolymer emulsion (INCI name), commercially available from Nippon LSC under the tradename Yodosol PUD (which also includes ethanol, 2-phenoyl-ethanol, and water in the emulsion).

The amount of sodium salt of the acrylates (co)polymer in the make-up removal compositions of the present invention generally ranges from about 0.4% to about 0.8%, and in some embodiments, from about 0.5% to about 0.7%, and in some other embodiments, from about 0.6% to about 0.65%, based on total weight of the composition.

In experiments wherein the sodium salt of an acrylates copolymer was substituted with an ammonium salt of an acrylates copolymer (specifically Ammonium Acryloyldimethyl taurate/VP Copolymer having the tradename Aristoflex AVC, available from Clariant), Applicants unexpectedly discovered that the resultant composition did not exhibit a rheological profile similar to inventive compositions as illustrated in Fig. 1. Use of sodium polyacrylate (e.g., commercially available from Cognis under the tradename Cosmedia SP), as well as Simulgel 600 (CTFA name: Acrylamide/Sodium Acryloyldimethyltaurate/Isohexadecane/Polysorbate-80), also yielded a rheological profile that does not fit with Fig. 1.

The polymeric stabilizing agent provides stability and consistency of texture, particularly during storage. It is selected however, so as not to affect the rheological properties of the inventive make-up removal compositions. Examples of polymeric stabilizing agents or stabilizing polymers include a fatty-chain gelling agent of the type which is a copolymer of a monoethylenic carboxylic acid containing 3 to 6 carbon atoms (or its anhydride) and of a long-chain acrylic ester. This type of water-soluble acrylic copolymer, which may be crosslinked and which is referred to in the art as a "fatty-chain gelling agent", is described in U.S. Patent 5,567,426 6 and EP-A-0 268 164. In this copolymer, the proportion of monomeric acid is preferably from 90 to 98% by weight and the proportion of monomeric ester is preferably from 10 to 2% by weight. The monomeric acid has the formula:

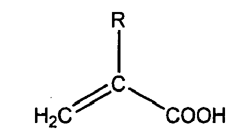

in which formula R represents H, a halogen, OH, a lactone or lactam radical, a group--C=N, or a C₁-C₃ alkyl radical. The preferred monomers are acrylic acid and maleic anhydride. The monomeric ester has the formula

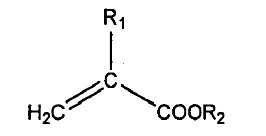

in which formula: R₁ is H, methyl or ethyl, and R₂ is a C₈-C₃₀ alkyl radical or a C₈-C₃₀ oxyalkylene radical. Monomeric esters which may be mentioned include: decyl, lauryl, stearyl, behenyl and melissyl acrylates and methacrylates. In some embodiments, the gelling agent is an acrylic or methacrylic acid copolymer, e.g., which includes the copolymers of C₁₀-C₃₀ alkyl acrylates and of acrylic or methacrylic acid or of their esters. Specific examples of the gelling agent are sold under the names Pemulen TR1®, Pemulen TR2® or Carbopol 1342® by the company Goodrich (CTFA name: Acrylates/C₁₀-C₃₀ Alkyl Acrylate Crosspolymer). The Pemulen products are known as polymeric emulsifiers that are predominantly high molecular weight polyacrylic acid polymers. They have a small lipophilic portion in addition to a large hydrophilic portion. This chemical structure allows them to function as primary emulsifiers.

The amount of the stabilizing polymer in the make-up removal compositions of the present invention generally ranges from about 0.001% to about 0.5%, and in some embodiments, from about 0.01% to about 0.2%, and in yet other embodiments from about 0.025% to about 0.075%, based on total weight of the composition.

Several types of hydrophobic wetting agents may be suitable for use in the present invention. One such type includes poly-α-olefins including the polyisobutenes (*e.g.,* hydrogenated polyisobutenes) sold under the name Permethyl 99 A, 101 A, 102 A, 104 A (n=16) and 106 A (n=38) by the company Presperse Inc., and the products sold under the name Arlamol HD (n=3) by the company ICI (n denoting the degree of polymerization), of hydrogenated or non-hydrogenated polydecene type, which are commercially available, for example, under the names Ethylflo by the company Ethyl Corp. and Arlamol PAO by the company ICI. include hydrogenated polydecene Other representative poly-α-olefins include hydrogenated and non-hydrogenated forms of isoeicosane and polybutene.

Yet other hydrophobic wetting agents include mono-, di- and tri-glycerides of C1-C30 carboxylic acids; *e.g.,* caprylic/capric triglyceride, PEG-6 caprylic/capric triglyceride, PEG-8 caprylic/capric triglyceride.

Other examples of hydrophobic wetting agents include alkoxylated fatty alcohols, which are ethers formed from the reaction of a fatty alcohol with an alkylene oxide, generally ethylene oxide or propylene oxide, *e.g.,* ethoxylated fatty alcohols which are adducts of fatty alcohols and polyethylene oxide. Specific examples of ethoxylated/propoxylated alcohols that may be suitable for use in the present invention include PPG-1 Beheneth-15, PPG-12 Capryleth-18, PPG-2-Ceteareth-9, PPG-4-Ceteareth-12, PPG-10-Ceteareth-20, PPG-1-Ceteth-1, PPG-1-Ceteth-5, PPG-1-Ceteth-10, PPG-1-Ceteth-20, PPG-2-Ceteth-1, PPG-2-Ceteth-5, PPG-2-Ceteth-10, PPG-2-Ceteth-20, PPG-4-Ceteth-1, PPG-4-Ceteth-5, PPG-4-Ceteth-10, PPG-4-Ceteth-20, PPG-5-Ceteth-20, PPG-8-Ceteth-1, PPG-8-Ceteth-2, PPG-8-Ceteth-5, PPG-8-Ceteth-10, PPG-8-Ceteth-20, PPG-2 C12-13 Pareth-8, PPG-2 C12-15 Pareth-6, PPG-4 C13-15 Pareth-15, PPG-5 C9-15 Pareth-6, PPG-6 C9-11 Pareth-5, PPG-6 C12-15 Pareth-12, PPG-6 C12-18 Pareth-11, PPG-3 C12-14 Sec-Pareth-7, PPG-4 C12-14 Sec-Pareth-5, PPG-5 C12-14 Sec-Pareth-7, PPG-5 C12-14 Sec-Pareth-9_{;} PPG-1-Deceth-6, PPG-2-Deceth-3, PPG-2-Deceth-5, PPG-2-Deceth-7, PPG-2-Deceth-10, PPG-2-Deceth-12, PPG-2-Deceth-15, PPG-2-Deceth-20, PPG-2-Deceth-30, PPG-2-Deceth-40, PPG-2-Deceth-50, PPG-2-Deceth-60, PPG-4-Deceth-4, PPG-4-Deceth-6, PPG-6-Deceth-4, PPG-6-Deceth-9, PPG-8-Deceth-6, PPG-14-Deceth-6, PPG-6-Decyltetradeceth-12, PPG-6-Decyltetradeceth-20, PPG-6-Decyltetradeceth-30, PPG-13-Decyltetradeceth-24, PPG-20-Decyltetradeceth-10, PPG-2-Isodeceth-4, PPG-2-Isodeceth-6, PPG-2-Isodeceth-8, PPG-2-Isodeceth-9, PPG-2-Isodeceth-10, PPG-2-Isodeceth-12, PPG-2-Isodeceth-18, PPG-2-Isodeceth-25, PPG-4-Isodeceth-10, PPG-12-Laneth-50, PPG-2-Laureth-5, PPG-2-Laureth-8, PPG-2-Laureth-12, PPG-3-Laureth-8, PPG-3-Laureth-9, PPG-3-Laureth-10, PPG-3-Laureth-12, PPG-4 Laureth-2, PPG-4 Laureth-5, PPG-4 Laureth-7, PPG-4-Laureth-15, PPG-5-*Laureth*-5*,* PPG-6-Laureth-3, PPG-25-Laureth-25, PPG-7 Lauryl Ether, PPG-3-Myreth-3, PPG-3-Myreth-11, PPG-20-PEG-20 Hydrogenated Lanolin, PPG-2-PEG-11 Hydrogenated Lauryl Alcohol Ether, PPG-12-PEG-50 Lanolin, PPG-12-PEG-65 Lanolin Oil, PPG-40-PEG-60 Lanolin Oil, PPG-1-PEG-9 Lauryl Glycol Ether, PPG-3-PEG-6 Oleyl Ether, PPG-23-Steareth-34, PPG-30 Steareth-4, PPG-34-Steareth-3, PPG-38 Steareth-6, PPG-1 Trideceth-6, PPG-4 Trideceth-6, and PPG-6 Trideceth-8.

Yet other hydrophobic wetting agents that may be suitable for use in the present invention include a water-soluble or water-dispersible polymer based on crosslinked or non-crosslinked homopolymers or copolymers comprising at least the acrylamido-2-methylpropanesulfonic acid (AMPS) monomer, in a form partially or totally neutralized with a mineral base other than ammonia, such as sodium hydroxide or potassium hydroxide. They are preferably totally neutralized or virtually totally neutralized, i.e., at least 90% neutralized. These AMPS polymers according to the invention may be crosslinked or non-crosslinked. Examples of water-soluble or water-dispersible AMPS homopolymers include crosslinked or non-crosslinked polymers of sodium acrylamido-2-methylpropanesulfonate, such as Simulgel 800 (CTFA name: Sodium Polyacryloyldimethyltaurate). Examples of AMPS copolymers (commercially available from Seppic) include AMPS/sodium acrylate copolymer sold under the name Simulgel EG (CTFA name: Acrylamide/Sodium Acryloyldimethyltaurate/Isohexadecane/Polysorbate-80)/

The amount of the hydrophobic wetting agent in the make-up removal compositions of the present invention generally ranges from about 0.4% to about 0.8%, and in some embodiments, from about 0.5% to about 0.7%, and in yet other embodiments from about 0.6% to about 0.65%, based on total weight of the composition.

Prepared mixtures of the sodium salt of the acrylates copolymer and the hydrophobic wetting agent may be commercially available. In this regard, mention may be made of Cosmedia SPL (i.e., sodium polyacrylate (and) Hydrogenated polydecene (and) PPG-5 Laureth 5, available from Cognis), and LUVIGEL EM (i.e., caprylic/capric triglyceride (and) sodium acrylates copolymer, commercially available from BASF).

The hydrophobic wetting agent, also referred to as a processing aid, is non-soluble and non-miscible in and with the aqueous phase, and functions to delay the wetting, swelling and hydration of the sodium salt of the acrylate copolymer. The schematic diagram below shows an inventive embodiment wherein the relatively large blackened spots represent LUVIGEL EM particles, and which surround oil droplets (represented by open circles) from which the Pemulin TR1® protrude. 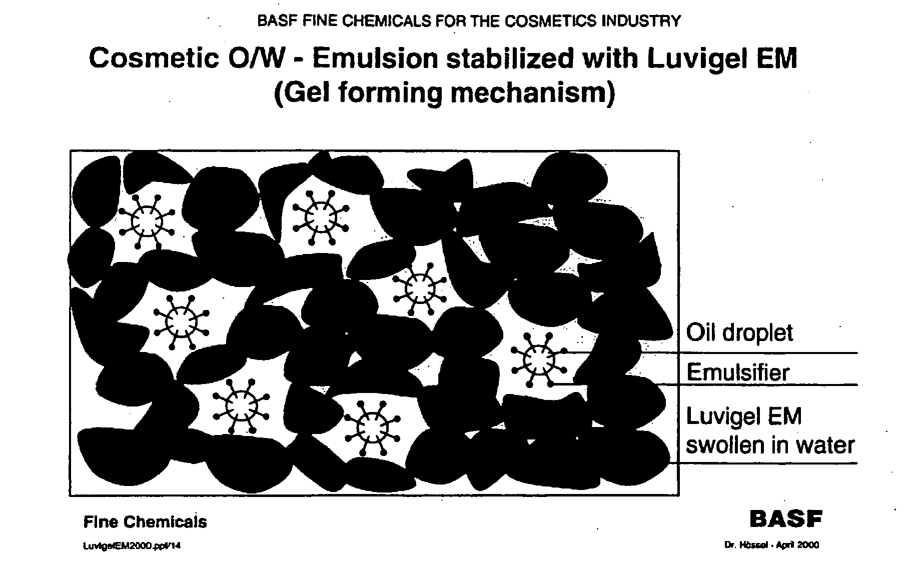 Without intending to be bound by theory, the acrylate backbone of the stabilizing polymer (e.g., the Pemulen TR1®) adheres to the oil phase, and the hydrophilic portions protrude into the water phase and form an interfacial barrier to coalescence, preventing the oil droplets from sticking together. The lipophilic portion of the Pemulen adsorbs at the oil-water interface, and the hydrophobic portion swells in the water forming a gel network around the oil droplets to provide excellent emulsion stability to the oils used in the present compositions. Otherwise, the composition is prone to what is referred to in the art as Ospwald ripening wherein oil droplets coalesce and degrade. As the make-up composition is rubbed onto the facial tissue, the matrix formed by the LUVIGEL EM is broken down (resulting in a decrease in viscosity), facial make-up (also referred to in the art as soil) is absorbed by the oil phase, and the matrix is then reformed (resulting in an increase in viscosity). This process is cyclic during the make-up removal process.

In a penultimate embodiment, the make-up removal composition contains about 1% to about 20% isopropyl myrstate, about 1% to about 20% isohexadecane, about 1.0% to about 10% cyclopentasiloxane, about 0.001% to about 0.5% acrylates/C10-C30 alkyl acrylate crosspolymer, about 0.4% to about 0.8% caprylic/capric triglyceride, and about 0.4% to about 0.8% sodium acrylates copolymer. It also contains an aqueious phase comprising water.

The aqueous phase of the make-up removal compositions contains water, typically in amounts ranging from about 50% to about 75% by weight of the composition. The aqueous phase may also contain cosmetically acceptable additives or adjuvants as well as cosmetic or dermatologic active agents. Representative additives and adjuvants include, for example, water-soluble or water-miscible solvents or co-solvents, humectants, moisturizers, colorants, fillers, preservatives, antioxidants (*e.g.,* EDTA, BHT, tocopherol), essential oils, fragrances, neutralizing or pH-adjusting agents (*e.g.,* triethylamine (TEA) and sodium hydroxide), conditioning or softening agents (*e.g.,* panthenol, allantoin and glycerin) and extracts such as botanical extracts. Additives and adjuvants may be present in the compositions in amounts generally ranging from about 0.01% to about 10% by weight. Examples of cosmetic active agents or dermatological active agents include sunscreen agents, free-radical scavengers, keratolytic agents, vitamins (*e.g.,* Vitamin E and derivatives thereof), anti-elastase and anti-collagenase agents, peptides, fatty acid derivatives, steroids, trace elements, extracts of algae and of planktons, enzymes and coenzymes, flavonoids and ceramides, -hydroxy acids and mixtures thereof, and enhancing agents. Active agents may be present in the compositions in amounts generally ranging from about 0.01% to about 10% by weight. These ingredients, additives, adjuvants and active agents alike, may be soluble or dispersible in whatever phase or phases are present in the mascara (i.e., aqueous and/or oil phase).

By way of representative example, suitable water-soluble or water-miscible solvents or co-solvents comprise short-chain monoalcohols, for example of C₁-C₄, for instance ethanol or isopropanol; diols or polyols, for instance ethylene glycol, 1,2-propylene glycol, 1,3-butylene glycol, hexylene glycol, diethylene glycol, dipropylene glycol, 2-ethoxyethanol, diethylene glycol monomethyl ether, triethylene glycol monomethyl ether and sorbitol.

Representative humectants include but are not limited to glycerin, diglycerin, triglycerin, polyglycerin, ethoxylated and propoxylated glycerols polypropylene glycol, polyethylene glycol, ethylene glycol, diethylene glycol, triethylene glycol, propylene glycol, dipropylene glycol, hexylene glycol, 1,3-butylene glycol and 1,4-butylene glycol. A preferred humectant is glycerin.

Suitable moisturizers include sodium lactate, mannitol, amino acids, hyaluronic acid, lanolin, urea, petroleum jelly and mixtures thereof. Other examples include polyols such as glycerin, diglycerin, triglycerin, polyglycerin, polyethylene glycol, ethylene glycol, diethylene glycol, triethylene glycol, propylene glycol, dipropylene glycol, hexylene glycol, 1,3-butylene glycol, 1,4-butylene glycol and sorbitol.

Colorants may be chosen from the lipophilic dyes, hydrophilic dyes, traditional pigments, and nacres usually used in cosmetic or dermatological compositions, and mixtures thereof. The coloring agent may have any shape, such as, for example, spheroidal, oval, platelet, irregular, and mixtures thereof. Pigments may optionally be surface-treated e.g., with silicones (e.g., inorganic pigments may be coated with simethicone), perfluorinated compounds, lecithin, and amino acids. The liposoluble dyes include, for example, Sudan Red, D&C Red 17, D&C Green 6, soybean oil, Sudan Brown, D&C Yellow 11, D&C Violet 2, D&C Orange 5, quinoline yellow and annatto. The water-soluble dyes are, for example, beetroot juice or methylene blue.

The pigments may be chosen from white pigments, colored pigments, inorganic pigments, organic pigments, coated pigments, uncoated pigments, pigments having a micron size and pigments not having a micron size. Among the inorganic pigments that may be mentioned are titanium dioxide, optionally surface-treated, zirconium oxide, zinc oxide, cerium oxide, chromium oxide, manganese violet, ultramarine blue, chromium hydrate, and ferric blue. Among the organic pigments which may be mentioned are carbon black, pigments of D&C type, lakes based on cochineal carmine, lakes based on barium, lakes based on strontium, lakes based on calcium, and lakes based on aluminum.

The nacreous pigments may, for example, be chosen from white nacreous pigments such as mica coated with titanium and mica coated with bismuth oxychloride, colored nacreous pigments such as titanium mica with iron oxides, titanium mica with, for example, ferric blue and/or chromium oxide, titanium mica with an organic pigment of the type mentioned above, as well as nacreous pigments based on bismuth oxychloride, interferential pigments, and goniochromatic pigments.

Examples of preservatives include alkyl para-hydroxybenzoates, wherein the alkyl radical has from 1, 2, 3, 4, 5 or 6 carbon atoms and preferably from 1 to 4 carbon atoms e.g., methyl para-hydroxybenzoate (methylparaben), ethyl para-hydroxybenzoate (ethylparaben), propyl para-hydroxybenzoate (propylparaben), butyl para-hydroxybenzoate (butylparaben) and isobutyl para-hydroxybenzoate (isobutylparaben). Mixtures of preservatives may be used, e.g., the mixture of methyl-paraben, ethylparaben, propylparaben and butylparaben sold under the name Nipastat by Nipa, and the mixture of phenoxyethanol, methylparaben, ethylparaben, propylparaben and butylparaben sold under the name Phenonip, also by Nipa.

The following examples are intended to further illustrate the present invention. They are not intended to limit the invention in any way. Unless otherwise indicated, all parts are by weight.

### EXAMPLE I -- INVENTIVE FORMULATION

An embodiment of the present invention is described in Table I.

**TABLE I**

| **PHASE** | **INGREDIENT** | **%** |
|---|---|---|
| A1 | DI Water | 66.600 |
| A1 | Glycerin | 5.000 |
| A1 | Methyl paraben | 0.300 |
| A1 | Caprylyl glycol | 0.500 |
| A2 | PEMULEN TR2 acrylates/C10-C30 alkyl acrylates crosspolymer | 0.050 |
| | | |
| B | Isohexadecane | 10.000 |
| B | Isopropyl myristate | 10.000 |
| B | Cyclopentasiloxane | 5.000 |
| | | |
| C | PURISOFT/botanical extract | 0.010 |
| C | LICHDODERM/botanical extract | 0.010 |
| C | EXFOLACTIVE EL/botanical extract | 0.010 |
| C | MOIST 24/botanical extract | 0.010 |
| C | GLYCOFILM/biosaccharide gum-4 | 0.200 |
| | | |
| D | LUVIGEL EM/caprylic/capric triglyceride (and) sodium acrylates copolymer | 2.500 |

The make-up removal composition was made according to the following procedure. Phase A1 ingredients (RM 511 S, 53, 633 B, and 71868) are added to a main kettle; heat to 75°C-80°C with low sweep mixing. Once dissolved, add Phase A2 (RM 53971) and mix with low homo and sweep until hydrated and uniform. Once Phase A1/A2 are uniform, cool to 65°C. Weigh Phase B (RM 53743, 1082, and 52390) in side kettle and prop mix moderately until phase is uniform while heating to 65°C. Add Phase B to A at 65°C and homo-37% & sweep mix-22%. Cool Phase A+B to 30°C and add pre-mixed Phase C (RM 1082 and 75440). Homo-37% and sweep-22% until viscosity develops. Keep mixing at same speeds and cool batch to 25°C.

### EXAMPLE II

### RHEOLOGICAL COMPARISON

Various experiments were conducted in which the embodiment of the present invention described in Example I (and which is a cream/polymer-based composition or system) was compared to other make-up removal compositions.

The three comparative compositions are referred to as the cream/surfactant-based system (the formula of which is shown in TABLE II), another cream/polymer or lotion-based composition (the formula of which is shown in TABLE II, and which is referred to as "comparative cream/polymer composition A"), and a second cream/polymer composition (the formula of which is shown in TABLE II, and which is referred to as "comparative cream/polymer composition B").

**TABLE II**

| **INCI US** | **(Inventive formulation - a cream/polymer composition)** | **(comparative cream/polymer composition B)** | **(comparative cream/polymer composition A)** | | **(comparative cream/surfactant- based composition)** |
|---|---|---|---|---|---|
| ALLANTOIN | | | | | 0.05 |
| DISODIUM EDTA | | | | | 0.2 |
| POTASSIUM PHOSPHATE | | | | | 0.1 |
| DIPOTASSIUM PHOSPHATE | | | | | 0.4 |
| HYDROLYZED OPUNTIA | 0.01 | 0.01 | | | |

| FICUS INDICA FLOWER EXTRACT (AND) ETHYLHEXYLGLYCERIN | | | | | |
|---|---|---|---|---|---|
| YELLOW 5 | | | 0.0002 | | |
| RED 40 | | | 0.0002 | | |
| ISOPROPYL MYRISTATE | 10 | | | | |
| ETHYLHEXL PALMITATE OIL | | 5 | 5 | | |
| PRUNUS ARMENIACA (APRICOT) KERNEL OIL | | | | | 17.59995 |
| ISOHEXADECANE | 10 | 5 | 5 | | |
| ETHYLHEXYL STEARATE | | 5 | 5 | | |
| FRAGRANCE | | | | | |
| FRAGRANCE | | | 0.15 | | |
| MICA (AND) BISMUTH OXYCHLORIDE (AND) IRON OXIDES | | | | | 0.003 |
| ACRYLATES/C10-30 ALKYL ACRYLATE CROSSPOLYMER | 0.05 | 0.001 | 0.001 | | |
| SCLEROTIUM GUM | | | | | 0.6 |
| CAPRYLIC/CAPRIC TRIGLYCERIDE (AND) SODIUM ACRYLATES COPOLYMER | 2.5 | 2 | 2 | | |
| BIOSACCHARIDE GUM-4 | 0.01 | 0.01 | | | |
| SODIUM DEHYDROACETATE | | | | | 0.3 |
| PHENOXYETHANOL | | | | | 0.3 |
| IMIDAZOLIDINYL UREA | | 0.2 | 0.2 | | |
| PROPYLPARABEN | | | | | 0.05 |
| METHYLPARABEN | 0.3 | 0.25 | 0.25 | | 0.25 |
| CYCLOPENTASILOXANE | 5 | | | | |
| BUTYLENE GLYCOL | | 10 | 10 | | |
| WATER | 66.6 | 72.349 | 72.2486 | | 74.77 |
| GLYCERIN | 5 | | | | |
| PENTYLENE GLYCOL | | | | | 1 |
| CAPRYLYL GLYCOL | 0.5 | 0.15 | 0.15 | | |
| PEG-7 GLYCERYL COCOATE | | | | | 0.91 |
| CETEARLY ALCOHOL (AND) COC-GLUCOSIDE | | | | | 2.73 |
| ZEA MAYS (CORN) OIL (AND) BETA-CAROTENE | | | | | 0.00005 |
| IMPERATA CYLINDRICA ROOT EXTRACT (AND) GLYCERIN (AND) PEG-8 (AND) CARBOMER | 0.01 | 0.01 | | | |
| GLYCERIN (AND) MORINGA PTERYGOSPERMA | 0.01 | 0.01 | | | |
| ASEED EXTRACT | | | | | |
| PROPYLENE GLYCOL (AND) CUCUMIS SATIVUS (CUCUMBER) FRUIT EXTRACT | | | | | 0.5 |
| BUTYLENE GLYCOL (AND) LITCHI CHINENSIS PERICARP EXTRACT | 0.01 | 0.01 | | | |
| TOCOPHERYL ACETATE | | | | | 0.23 |
| **TOTAL** | **100.0** | **100.0** | **100.0** | | **100.0** |

The experiments were conducted using a rheometer Prorheo Model R 180, in accordance with the manufacturer's instructions. The following parameters were employed: temperature 25°C +/-1°C; shear program -- start shear rate (0.0 1/seconds), mid point shear rate (200.0 1/seconds), and end shear rate (0.0 1/seconds); and sensor system Proreheo TV spindle #3. The experimental procedures were as follows: fill Prorheo TV cup with 28 grams of make-up removal composition; equilibrate sample to 25°C +/-1°C in controlled temperature bath for 10 minutes; insert TV# 3 sensor probe into TV sample cup start rheo program software; and when test is complete, store/print rheogram using Rheo software package.

The results of the experiments are illustrated in Figs. 1-3. The rheological comparison between the inventive composition and the cream/surfactant-based composition is shown in Fig. 1; the rheological comparison between the inventive composition and the comparative cream/polymer composition A is shown in Fig. 2; and the rheological comparison between the inventive composition and the comparative cream/polymer composition B is shown in Fig. 3.

### EXAMPLE III - Consumer Performance Testing

The objective of this experiment was to obtain the opinions and perceptions of consumers with respect to the general aesthetics, comfort and irritation of an embodiment of the present invention and a commercially available make-up removal composition which is based on silicone oil, on consumers with and without waterproof make-up.

The methods entailed flash consumer cross-overs, semi-qualitative interviews with closed questions. Twenty (20) women were recruited to test both the inventive composition and the commercial composition without make-up applied to the face. The ages of the women range from 18-65 years. They used waterproof make-up remover to remove waterproof make-up 5-7 times per week. Thirteen (13) of the 20 women were then recruited for further testing of the compositions with waterproof mascara, foundation, and lipstick applied to the face. Evaluation was conducted immediately upon application of the make-up removal composition.

The make-up composition of the present invention used in this study is described in Example I, above. The formula of the comparative composition (hereinafter the "silicone oil" composition) is set forth below.

**TABLE III**

| **INCI US** | **AMOUNT (IN % BY WT)** |
|---|---|
| POTASSIUM PHOSPHATE | 0.0534 |
| DIPOTASSIUM PHOSPHATE | 0.1602 |
| SODIUM CHLORIDE | 0.4806 |
| ISOHEXADECANE | 18.64 |
| FRAGRANCE | 0.006675 |
| FRAGRANCE | 0.00267 |
| QUATERNIUM-15 (and) BENZALKONIUM CHLORIDE | 0.267 |
| BENZYL ALCOHOL | 0.1165 |
| CYCLOPENTASILOXANE | 27.8435 |
| HEXYLENE GLYCOL | 0.267 |
| WATER | 51.895455 |
| POLOXAMER 184 | 0.267 |

Regarding the embodiment of the present invention, satisfaction was determined to be greater than average because 12 of the 13 women were satisfied, ranging from somewhat to very satisfied, whereas only one of the 13 women was somewhat dissatisfied. The women determined that the inventive composition had many strengths, including a pleasant texture with a non-greasy product feel, just the right consistency, ease of application, non-greasy skin feel, no sticky skin feel, clean skin feel, easy make-up removal and product removal, effective face make-up removal, effective lipstick removal, and comfortable skin feel. On the other hand, no weaknesses were accumulated by the women. Regarding irritation, 4 out of 20 women experienced slight to moderate irritation in the face and eye areas without make-up. Three (3) out of 13 women experienced slight irritation in the face and eye area with make-up.

Regarding the silicone oil composition, satisfaction was determined to be less than average. Nine (9) of the 13 women were satisfied, ranging from somewhat to very satisfied, and one woman was neither satisfied nor dissatisfied. Three (3) of the 13 women were dissatisfied, ranging from somewhat to very dissatisfied. According to the women, the strengths of the silicone oil composition included a pleasant texture, ease of application, no sticky skin feel, easy make-up removal, effective eye make-up removal, effective face make-up removal, effective lipstick removal and comfortable skin feel. On the other hand, the women determined that the silicone oil composition had several weaknesses as well, including, somewhat thin product consistency, very to slightly greasy product feel, not the right amount of moisture, and requiring too much rubbing (ranging from slightly, to excessive). Regarding irritation, one of the 20 women experienced very slight irritation in the eye area without make-up, and 3 of the 13 experienced slight irritations in the face and eye area with make-up.

Overall, inventive composition was accepted by the women at above average, with 12 out of 13 women finding themselves somewhat to very satisfied, while the silicone oil composition was accepted by the women at below average, with 9 out of 13 women somewhat to very satisfied, and 3 out of 13 somewhat to very dissatisfied.

When tested with make-up on the face, both test products were found to have pleasant textures, left skin feeling not sticky, easily removed make-up and effectively removed face make-up and lipstick. However, the silicone oil composition was found to remove eye make-up effectively, but with a somewhat thin product consistency, very to slightly greasy product feel and left skin without the right amount of moisture.

The amount of time it took each panelist to remove her make-up was recorded while using both products. The average length of time was similar, with the inventive composition requiring an average time of 99 seconds, and the silicone oil product requiring an average time of 96 seconds.

Regarding irritation, both test products were easy to apply, left skin feeling comfortable, and caused slight irritation to the eyes and face both with and without make-up applied. The number of instances of irritation and degree of irritation as described by the panelist were similar (with and without applied make-up).

Women observed that their skin was left with a slight to heavy oily/greasy residue when the silicone oil composition was used without make-up on the face, but that the residue was not as apparent when using the comparative composition with make-up on the face. The silicone oil composition was also found to require harsher rubbing than the inventive composition to remove make-up from the face.

Overall, preference of the panelists was in favor of the inventive composition, with 9 out of 13 women preferring the inventive composition over the silicone oil composition. Women observed liking that the inventive composition left their skin feeling less oily/greasy (4 mentions), more moisturized, cleaner, and easier to apply and remove make-up on the face and eyes (3 mentions each).

### EXAMPLE IV

The purpose of this experiment was to compare the efficacy of the embodiment of the present invention described in Example 1 to the following make-up remover standards, namely: 1) the cream/surfactant based composition shown in TABLE II; 2) a mineral oil based composition, the formula of which is shown in TABLE IV below; 3) the silicone oil-based composition described in TABLE III; and the cream/polymer composition B shown in TABLE II (which as shown in Fig. 3, does not exhibit the rheological profile which is a feature of the inventive compositions. The formulas of comparative compositions 1, 3 and 4 are described in Tables above. The formula of comparative composition 2 is set forth in TABLE IV below.

**TABLE IV**

| **INGREDIENT** | **% (BY WT.)** |
|---|---|
| Dicapryl carbonate | 2.0000 |
| Isopropyl myristate | 22.0000 |
| Kaydol white mineral oil | 64.4500 |
| C12-15 Alkyl benzoate | 5.0000. |
| Caprylyl glycol | 0.1000 |
| Uniox GT-20IS | 5.0000 |
| Emalex 300DI-ISEX | 0.5000 |
| Polysorbate 85 | 0.6500 |
| Fragrance | 0.3000 |

The experimental procedure was as follows. The following products were applied to the volar forearm and dried for 15 minutes on 2 x 2cm test sites as follows: Site A - Le stylo waterproof eyeliner -evenly coated; Site B - Eva Longoria Caramel lipstick -evenly coated; Site C - HIP Liquid foundation -8µL evenly spread; and Site D - Lip Duet lip liner
- evenly coated. Make-up removers were applied to each test site with moderate finger pressure along with a circular motion. Product was then removed with cotton swab. The time taken to remove products from the test site was recorded. The results are tabulated below.

**TABLE IV (TEST SUMMARY)**

| **PRODUCT:** | **INVENTIVE COMPOSITION** | **AMOUNT: 20**µ**L** |
|---|---|---|
| **SITE REMOVAL TIME COMMENTS** | A = 11 seconds | |
| | | |
| | B = 7 seconds | |
| | C = 12 seconds | |
| | D = 10 seconds | |
| | Mixed quickly into dried make-up, did not | |
| | run, pick-up was easy on the cloth, minimal | |
| | to no residue | |
| **PRODUCT: SITE REMOVAL TIME COMMENTS** | **INVENTIVE COMPOSITION** | **AMOUNT: 40**µ**L.** |
| | | |
| | A = 12 seconds | |
| | B = 9 seconds | |
| | C = 6 seconds | |
| | D = 13 seconds | |
| | Mixed quickly into dried make-up, did not | |
| | run, pick-up was easy on the cloth, minimal | |
| | to no residue | |
| **PRODUCT:** S**ITE** A = **REMOVAL TIME COMMENTS** | **Cream-Surfactant composition** | **AMOUNT: 40**µ**L.** |
| | 28 seconds | |
| | B = 13 seconds | |
| | C = 28 seconds | |
| | D = 30 seconds | |
| | Mixed quickly into dried make-up, did not | |
| | run, pick-up was easy on the cloth, minimal | |
| | to no residue | |
| **PRODUCT: SITE REMOVAL TIME COMMENTS** | **Mineral Oil-based composition** | **AMOUNT: 40**µ**L** |
| | | |
| | A = 12 seconds | |
| | B = 9 seconds | |
| | C = 28 seconds | |
| | D = 29 seconds | |
| | Mixed well into certain dried make-up, did | |
| | not run, pick-up was easy on the cloth, | |
| | slight to minimal residue | |
| **PRODUCT: SITE REMOVAL** | **Silicone Oil-based composition** | **AMOUNT: 40**µ**L** |
| | | |
| | A = 46 seconds | |
| **TIME COMMENTS** | B = 32 seconds | |
| | C = 28 seconds | |
| | D = 29 seconds | |
| | Did not mix well into dried make-up, | |
| | product ran, pick-up difficult on cloth, | |
| | moderate to high residue | |
| **PRODUCT: SITE REMOVAL TIME COMMENTS** | **Comparative cream/polymer composition B** | **AMOUNT: 40**µ**L** |
| | A = 29 seconds | |
| | B = 27 seconds | |
| | C = 31 seconds | |
| | D = 36 seconds | |
| | Did not mix well into dried make-up, did | |
| | not run, pick-up wasn't easy on the cloth, | |
| | mild to moderate residue | |

It was determined that the inventive make-up removal composition was very efficient in terms of time and performance at all product sites compared to the other make-up removers. It was also determined that the inventive composition left the skin feeling soft and non-greasy as compared to some of the comparative compositions that left an oily feel. The texture of inventive composition further allowed for a more controlled make-up removal because it stayed on the make-up site while removing. It was observed that the liquid make-up removers were more prone to run-off from the make-up site.

All publications cited in the specification, both patent publications and non-patent publications are indicative of the level of skill of those skilled in the art to which this invention pertains. All these publications are herein incorporated by reference to the same extent as if each individual publication were specifically and individually indicated as being incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the appended claims.

## Claims

1. A make-up removal composition, comprising a mineral oil-free oil phase; a sodium salt of an acrylate copolymer; a stabilizing polymer; and a hydrophobic wetting agent, wherein said composition exhibits a variable shear stress response to increasing shear rates comprising 20, 40, 80 and 150 1/seconds.

2. The make-up removal composition of claim 1, wherein said oil phase comprises a straight or branched chain hydrocarbon or ester thereof.

3. The make-up removal composition of claim 2, wherein said straight or branched chain hydrocarbon or ester thereof comprises isohexadecane, isopropyl myrstate, octyl palmitate or octyl stearate.

4. The make-up removal composition of claim 2, wherein said oil phase comprises isohexadecane and isopropyl myrstate.

5. The make-up removal composition of claim 1, wherein oil phase comprises about 20 to about 30% by weight of said composition.

6. The make-up removal composition of claim 1, wherein said sodium salt of an acrylate copolymer comprises sodium acrylates copolymer.

7. The make-up removal composition of claim 1, wherein said stabilizing polymer comprises a fatty-chain gelling agent comprising a copolymer of a monoethylenic carboxylic acid containing 3 to 6 carbon atoms, or an anhydride thereof, and of a long-chain acrylic ester.

8. The make-up removal composition of claim 7, wherein said gelling agent comprises a copolymer of a C₁₀-C₃₀ alkyl (meth)acrylate.

9. The make-up removal composition of claim 8, wherein said gelling agent comprises an acrylates/C10-30 alkyl acrylate crosspolymer.

10. The make-up removal composition of claim 9, wherein said hydrophobic wetting agent comprises a mono-, di-, or tri-glyceride of a C1-C30 carboxylic acid.

11. The make-up removal composition of claim 1, comprising about 1% to about 20% isopropyl myrstate, about 1% to about 20% isohexadecane, about 1.0% to about 10% cyclopentasiloxane, about 0.001% to about 0.5% acrylates/C10-C30 alkyl acrylate crosspolymer, about 0.4% to about 0.8% caprylic/capric triglyceride, and about 0.4% to about 0.8% sodium acrylates copolymer.

12. The make-up removal composition of claim 1, further comprising a cosmetic additive or adjuvant.

13. The make-up removal composition of claim 1, further comprising a cosmetic or dermatologic active ingredient.

14. A method of removing make-up from facial tissue or skin, comprising applying to facial tissue a make-up removal composition, comprising a mineral oil-free oil phase; a sodium salt of an acrylate copolymer; a stabilizing polymer; and a hydrophobic wetting agent, wherein said composition exhibits a variable shear stress response to increasing shear rates comprising 20, 40, 80 and 150 1/seconds.
